# EUROPÄISCHE PATENTSCHRIFT

(11) **EP 0 837 877 B1**
(45) Veröffentlichungstag und Bekanntmachung des Hinweises auf die Patenterteilung: **16.08.2001**
(21) Anmeldenummer: 96920048.4
(22) Anmeldetag: 22.05.1996
(51) Int. Cl.: C07K 5/06

(54) **L-ASPARTYL-L-THIENYLALANINMETHYLESTER, VERFAHREN ZU DEREN HERSTELLUNG UND VERWENDUNG ALS SÜSSSTOFF**
L-ASPARTYL-L-THIENYLALANINE METHYL ESTERS, PROCESS FOR PRODUCING THEM AND THEIR USE AS SWEETENERS
L-ASPARTYL-L-THIENYLALANINEMETHYLESTERS, LEURS PROCEDES DE PRODUCTION ET LEUR UTILISATION COMME EDULCORANTS

(30) Priorität: 26.05.1995 AT 886955
(43) Veröffentlichungstag der Anmeldung: 29.04.1998
(73) Patentinhaber: PharmaCon Forschung und Beratung GmbH, 4780 Schärding (AT)
(72) Erfinder: NOE, Christian, D-60596 Frankfurt (DE); WEIGAND-BECKER, Antje, D-60435 Frankfurt (DE)
(74) Vertreter: Berger, Erhard, Dr.
(86) Internationale Anmeldenummer: NL9600207
(87) Internationale Veröffentlichungsnummer: WO9637507

(56) Entgegenhaltungen:
- EP-A- 0 284 084
- EP-A- 0 316 725
- US-A- 4 692 513

## Beschreibung

Künstliche Süssstoffe finden in einer Vielzahl von Nahrungsmitteln Anwendung, wobei Saccharin, Cyclamat, Acesulfam-K und Aspartam zu den bekanntesten verwendeten künstlichen Süssungsmitteln zählen. Der Einsatz von Saccharin und Cyclamat gab jedoch immer wieder Anlass zu Diskussionen über ihre gesundheitliche Unbedenklichkeit und führte so zur Entwicklung neuer Süssstoffe mit hoher Süsskraft. So finden in jüngster Zeit die Süssstoffe Aspartam und Acesulfam-K zunehmende Verbreitung als Lebensmittelzusatzstoff, in deren Folge zahlreiche Untersuchungen über ihre Eigenschaften und Einsatzmöglichkeiten durchgeführt wurden. Da Acesulfam-K in seinen geschmacklichen Qualitäten an Aspartam nicht heranreicht, ist Aspartam das bekanntere und häufiger eingesetzte künstliche Süssungsmittel. Da jedoch manche Personen die Einnahme von Aspartam nicht vertragen, etwa bei Stoffwechselerkrankungen die im Zusammenhang mit Phenylalanin stehen, liegt die eigentliche Problemlösung auf dem Gebiet der künstlichen Süssstoffe noch immer auf der Entwicklung neuer Produkte, die gut verträglich sind und eine hohe Süsskraft aufweisen. Aufgabe der vorliegenden Erfindung war es demnach, Verbindungen zu finden, die die Nachteile der bisher bekannten Süssstoffe nicht aufweisen und die eine gegenüber dem Stand der Technik gleich grosse oder erhöhte Süsskraft aufweisen. Aus dem Stand der Technik, etwa aus J. Med. Chem. 1990, 33, Seiten 1676 - 1682, sind beispielsweise Verbindungen bekannt, bei welchen die Phenylalaningruppe von Aspartam ersetzt wurde. Es wurden dabei 8 L-Aspartyl-dipeptide, die von heterocyclischen Glycinestern abgeleitet wurden hergestellt und untersucht. Dabei wurde jedoch festgestellt, dass die β-Thienylalaninfenchylesterverbindungen (Verbindung 14) keine Süsskraft besitzen.

Unerwarteterweise wurden nun Verbindungen gefunden, bei welchen die Phenylalaningruppe des Aspartams durch eine Thienylalaningruppe ersetzt ist und die eine gegenüber Aspartam erhöhte oder zumindest gleich grosse Süsskraft aufweisen.

Gegenstand der vorliegenden Erfindung sind demnach L-Aspartyl-L-thienylalaninmethylester der Formel 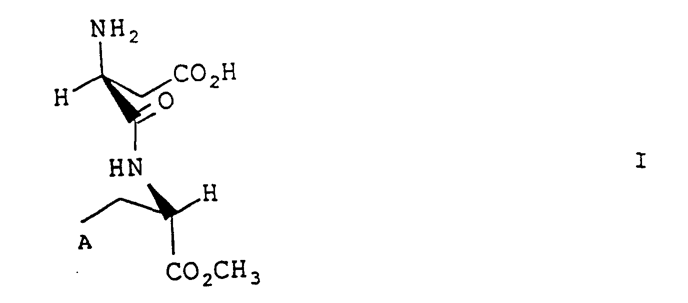 in der A einen Rest der Formel 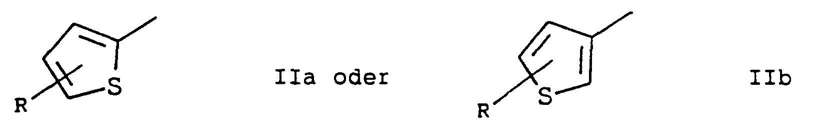 bedeutet, worin R H oder CH₃ sein kann.

A bedeutet somit in der Formel I einen Rest der Formel IIa oder IIb der gegebenenfalls durch einen Methylrest substituiert sein kann.

Beispiele dafür sind 2'-Thienyl, 3'-Thienyl, 2'-(5'-Methyl)thienyl, 2'-(4'-Methyl)thienyl oder 3'-(5'-Methyl)thienyl. Bevorzugt bedeutet X einen 2'-Thienyl-, 3'-Thienyl- oder 2'-(5'-Methyl) thienylrest.

"Es sei bemerkt dass in EP-A-316725 N-geschützte Aspartyl-Thienylalanin-verbindungen beschrieben sind, doch Verwendung als Süssstoff der nicht-geschützten Verbindungen wird nicht erwähnt. Andererseits sind in EP-A-284084 und US-A-4692513 ungeschützte Aspartyl-derivate beschrieben die sich als süss erweisen. Die darin genannte Verbindungen sind strukturell aber sehr von den jetzigen erfindungsgemässen Verbindungen unterschieden.

Die Herstellung der erfindungsgemässen Verbindungen kann auf chemischem oder enzymatischem Weg erfolgen. Bevorzugt werden konventionelle chemische Verfahren angewandt. So wird beispielsweise L-Asparaginsäure, deren α-Aminogruppe und β-Carboxylgruppe geschützt sind, durch Aktivierung der α-Carboxylgruppe in ein reaktives Derivat überführt, das mit dem Hydrochlorid der entsprechenden Thienylalaninverbindung kondensiert wird, worauf im Anschluss an die Kondensation die Abspaltung der Schutzgruppen erfolgt.

Bevorzugte Schutzgruppen sind dabei beispielsweise für die Amino-Funktion die Benzyloxycarbonylgruppe (Z) oder die tert. Butyloxycarbonylgruppe (Boc), sowie für die β-Carboxylgruppe der tert.-Butylrest (t-Bu). Die Abspaltung der beiden Schutzgruppen kann dann in einem Schritt acidolytisch (HBr/CH₃COOH oder HF) erfolgen.

Anstelle der oben angeführten Schutzgruppen bzw. Abspaltungsmethoden können jedoch auch andere aus dem Stand der Technik der Peptidsynthesen bekannte Schutzgruppen oder Abspaltungsmethoden eingesetzt werden.

Die Aktivierung der α-Carboxylgruppe kann ebenfalls nach einer aus dem Stand der Technik bekannten Methoden, beispielsweise nach der Azid-, der gemischten Anhydrid-, der Aktivester- oder der Carbodiimidmethode erfolgen.

Zur Herstellung der erfindungsgemäßen Verbindungen wird somit zuerst die α-Carboxylgruppe der L-Asparaginsäure, deren α-Aminogruppe und β-Carboxylgruppe geschützt sind, beispielsweise die α-Carboxylgruppe von (S)-N-Benzyloxycarbonylasparaginsäure-β-tert.butylester, aktiviert, etwa durch Reaktion mit Trichlorphenol in Gegenwart von Dicyclohexylcarbodiimid (DCC). Dazu wird eine Lösung der Asparaginsäure und Trichlorphenol, das bevorzugt in leichtem Überschuss eingesetzt wird, bei +10 bis -10°C mit DCC versetzt und 2 bis 10 Stunden gerührt. Als Lösungsmittel eignet sich dabei beispielsweise Ethylacetat. Anschließend wird auf etwa 15 bis 30°C erwärmt, noch 5 bis 30 Minuten gerührt, der ausgefallene Harnstoff abfiltriert, mehrmals mit Ethylacetat gewaschen und das Filtrat im Vakuum vom Lösungsmittel befreit. Das zurückbleibende Öl wird sodann über Kieselgel mit einem Petrolether/Ether-Gemisch geflasht.

Das Edukt für diesen Reaktionsschritt, (S)-N-Benzyloxycarbonylasparaginsäure-β-tert.butylester kann beispielsweise analog E. Wünsch, A. Zwick, Z.physiol. Chem. 328 (1962) 235 hergestellt werden.

Anschliessend erfolgt die Kupplung mit dem Hydrochlorid der entsprechenden Thienylalaninverbindung in Gegenwart eines Säurefängers, wie etwa Triethylamin, Pyridin u.a.

Bevorzugt wird dabei das im vorangegangenen Schritt erhaltene Produkt in einem geeigneten, wasserfreien Lösungsmittel, wie etwa DMF, gelöst und bei +5 bis -10°C in Gegenwart des Säurefängers mit der Hydrochloridverbindung noch mehrere Tage, bevorzugt 2 bis 5 Tage, gerührt.

Die Isolierung des Kupplungsproduktes erfolgt bevorzugt durch Extraktion beispielsweise mit Ether und Eiswasser, Trocknung der organischen Phase, etwa mit Natriumsulfat, Magnesiumsulfat und Abdestillieren des Lösungsmittels. Anschliessend erfolgt wiederum das Flashen über Kieselgel. Das Hydrochlorid des (S)-3-(2'-Thienyl)alaninmethylester ist beispielsweise aus J. Dunn. J. biol. Chem. 234 (1959), S. 802 bekannt, (S)-3-(3'-Thienyl)alanin ist beispielsweise in K. Dittmer et al., J. Am. Chem. soc. 71 (1949), S. 1201 beschrieben.

Im Anschluss an die Kupplung erfolgt die Schutzgruppenabspaltung etwa durch Zusatz von HBr und Eisessig, worauf das gewünschte Endprodukt in Salzform isoliert werden kann.

Die Abspaltung erfolgt dabei bei einer Temperatur von 15 bis 30°C, bevorzugt bei Raumtemperatur, unter tropfenweiser Zugabe einer Lösung von HBr in Eisessig zu einer Eisessig-Lösung des im vorangegangenen Schrittes erhaltenen Produktes und unter 1 bis 4-stündigem Rühren.

Anschliessend werden die Lösungsmittel abdestilliert, der Rückstand in Ether oder Dioxan aufgenommen und das ausfallende Produkt abfiltriert und im Vakuum getrocknet. Um das gewünschte Endprodukt aus dem so erhaltenen Hydrobromid zu isolieren, wird die Hydrobromidverbindung in Wasser gelöst und mit einer Base, beispielsweise mit einem basischen Ionentauscher, wie etwa mit Amberlite, versetzt und 10 bis 60 Minuten bei 30 bis 60°C gerührt. Der Feststoff wird dann abfiltriert, mit Wasser gewaschen und das Eluat, sowie die Waschlösung im Vakuum so stark eingeengt, dass das entsprechende Endprodukt ausfällt, das sodann abfiltriert und getrocknet wird.

Ein weiterer Gegenstand der vorliegenden Erfindung ist demnach ein Verfahren zur Herstellung von Verbindungen der Formel 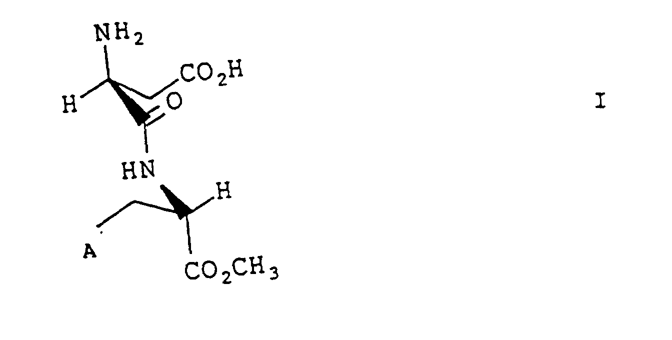 in der A einen Rest der Formel 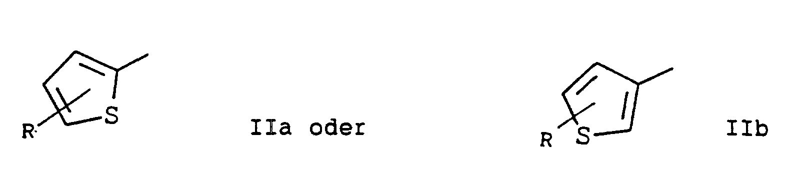 bedeutet, worin R H oder CH₃ sein kann, das dadurch gekennzeichnet ist, dass eine Verbindung der Formel 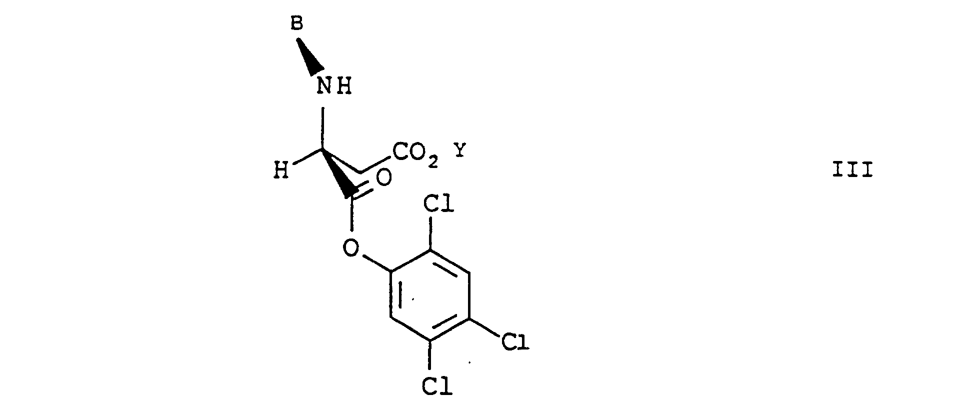 in der B eine Aminoschutzgruppe und Y eine Carboxyschutzgruppe bedeutet, in Gegenwart eines Säurefängers mit einer Verbindung der Formel 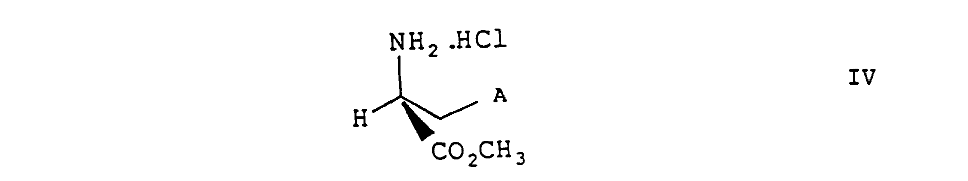 in der A obige Bedeutung hat, zu einer Verbindung der Formel 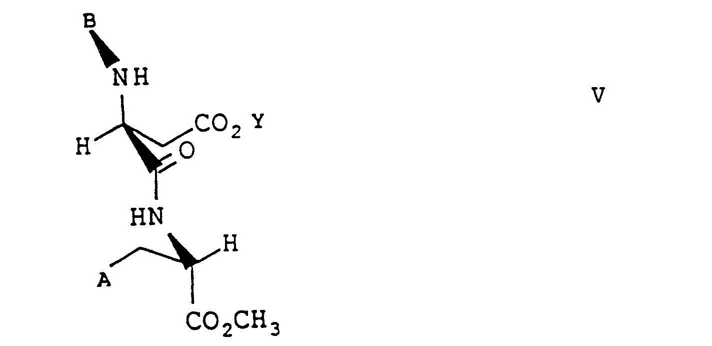 umgesetzt wird, in der B und Y obige Bedeutung haben, worauf nach Abspaltung ender Schutzgruppen auf acidolytischem Weg, die Isolierung der entsprechenden Verbindung der Formel I aus dem so gewonnenen Salz mittels einer Base erfolgt.

Die erfindungsgemässen Verbindungen der Formel I zeichnen sich durch eine gegenüber dem Stand der Technik verbesserte oder gleichgrosse Süsskraft selbst bei Zugabe geringer Mengen aus und eignen sich deshalb zur Verwendung als künstlicher Süssstoff.

Ein weiterer Gegenstand der vorliegenden Erfindung ist demnach die Verwendung von Verbindungen der Formel 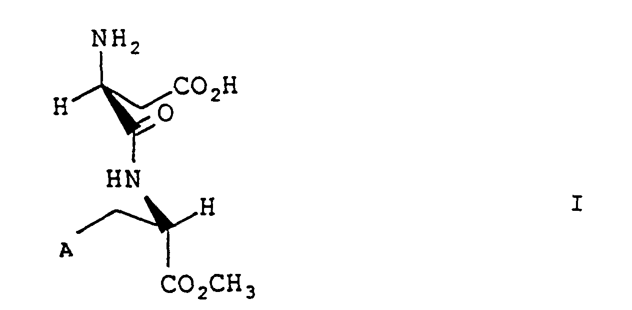 in der A einen Rest der Formel 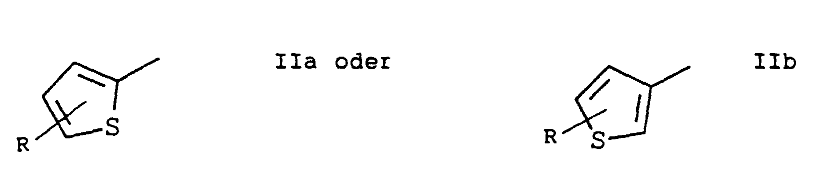 bedeutet, worin R H oder CH₃ sein kann, als künstlicher Süssstoff.

### Beispiel 1

### Herstellung von α-Aspartyl-L-(2-thienyl)alanin, Methylester (1)

### 1.1. (S)-N-Benzyloxycarbonyl-asparaginsäure-β-tert.-butyl-α-2,4,5-trichlorphenylester (4)

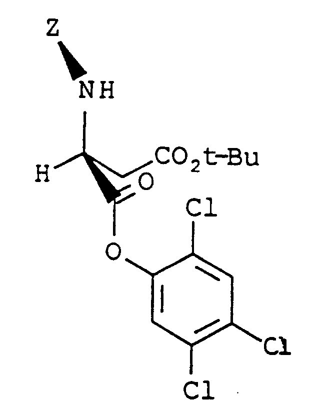

Eine Lösung von 7.5 g (23.2 mmol) (S)-N-Benzyloxycarbonyl-asparaginsäure-β-tert.butyl-ester und 5.05 g (25.6 mmol) Trichlorphenol in 60 ml Ethylacetat wurde bei 0 °C mit 4.74 g (23.3 mmol) DCC versetzt und im Eisbad 4 h lang gerührt.
Nach weiteren 15 min Rühren bei Raumtemperatur wurde der ausgefallene Harnstoff abfiltriert, mehrmals mit Ethylacetat gewaschen und das Filtrat im Vakuum von Lösungsmittel befreit. Das zurückbleibende Öl wurde über 260 g Kieselgel mit Petrolether (40 - 60 °C) /Ether (Gradient von 9:1 bis 7:3) geflasht.
Ausbeute :
8.3 g, (71%), farbloses Öl
R_{f} = 0.28 (Petrolether:Ether = 9:1)
[α]²³_{D}= -6°, (c = 1.0, CH₂Cl₂)
^{**1**}**H-NMR** (CDCl₃): δ = 7.56 (s; 1H, CH), 7.42 - 7.32 (m; 6H, C₆H₅, CH), 5.91 (d; 1H, NH), 5.18 (s; 2H, OCH₂), 4.92 (m; 1H, CH), 3.14 (dd; 1H, CH von -CH₂-), 2.92 (dd; 1H, CH von -CH₂-), 1.48 (s; 9H, CH₃).
^{**13**}**C-NMR** (CDCl₃): δ = 169.87 (s; COO), 168.39 (s; COO), 155.97 (s; CONH), 145.44 (s; C), 135.99 (s; C), 131.55 (s; C), 130.87 (d; CH), 128.56 (d; CH), 128.29 (d; CH), 128.13 (d; CH), 125.86 (s; C), 125.11 (d; CH), 82.40 (s; OC), 67.36 (t; OCH₂), 50.58 (d; CH), 37.68 (t; -CH₂-), 28.03 (q; CH₃).

### 1.2. L-β-tert.Butyl-N-carbobenzoxy-α-aspartyl-L-(2-thienyl)alanin; Methylester (5)

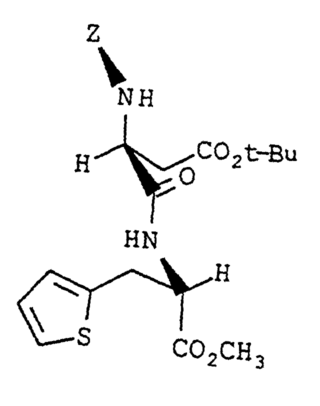

8.0 g (15.91 mmol) 4 werden in 50 ml wasserfreiem DMF gelöst und bei 0 °C mit 3.53 g (15.91 mmol) (S)-3-(2'-Thienyl)-alanin, Methylester, Hydrochlorid und 2.20 ml (15.91 mmol) Triethylamin versetzt und 3 Tage bei 0 - 4°C gerührt. Die Reaktionslösung wurde anschliessend Eiswasser und Ether verteilt, die wässrige Phase mehrmals mit Ether extrahiert, die vereinigten organischen Phasen mit Natriumsulfat getrocknet und das Lösungsmittel abdestilliert. Das so erhaltene Rohprodukt wurde über 220 g Kieselgel mit Petrolether:Ether (Gradient von 6:4 aus 1:2) geflasht.
Ausbeute :
5.7 g (73 %), farbloser Feststoff, Smp: 56 - 57 °C
R_{f} = 0.36 (Petrolether:Ether = 1:2), [α]²⁵_{D} = + 41.5 °, (c = 0.89, CH₂Cl₂)
^{**1**}**H-NMR** (CDCl₃): δ = 7.30 - 7.25 (m; 5H, C₆H₅), 7.07 (dd; 1H, Th-H-3), 7.02 (bs; 1H, CONH), 6.85 (dd; 1H, Th-H-4), 6.78 (dd; 1H, Th-H-5), 5.90 (d; 1H, CONH), 5.06 (s; 2H, OCH₂), 4.77 (m; 1H, CH), 4.50 (m; 1H, CH), 3.67 (s; 3H, OCH₃), 3.28 (m; 2H, -CH₂-), 2.85 (dd; 1H, CH von -CH₂-), 2.55 (dd; 1H, CH von -CH₂-), 1.36 (s; 9H, CH₃).
^{**13**}**C-NMR** (CDCl₃): δ = 170.99 (s; CO), 170.81 (s; CO), 170.31 (s; CO), 156.51 (s; CONH), 137.02 (s; C), 136.08 (s; C), 128.06 (d; CH), 128.23 (d; CH), 128.06 (d; CH), 127.07 (d; CH), 126.99 (d; CH), 124.86 (d; CH), 81.82 (s; OC), 67.21 (t; OCH₂), 53.35 (q; OCH₃), 52.45 (d; CH), 51.09 (d; CH), 37.28 (t; -CH₂-), 31.85 (t; -CH₂-), 27.99 (q; CH₃).

| | | | |
|---|---|---|---|
| C₂₄H₃₀N₂O₇S (490.58): | | | |
| Ber. | C 58.76 | H 6.16 | N 5.71 |
| Gef. | C 58.55 | H 6.12 | N 5.66 |

### 1.3. N-L-α-Aspartyl-L-(2-thienyl)alanin, Methylester, Hydrobromid (6)

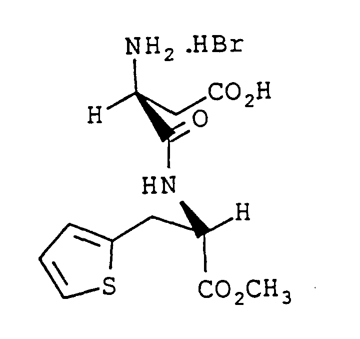

Eine Lösung von 5.0 g (10.2 mmol) 5 in 20 ml Eisessig wurde bei Raumtemperatur tropfenweise mit 15 ml (61.2 mmol) einer 4.1 M (33 %) Lösung von HBr in Eisessig versetzt und 2 h gerührt.

Die Lösungsmittel wurden im Wasserstrahlvakuum abdestilliert und der ölige Rückstand mit 100 ml Ether digeriert. Dabei fiel ein farbloses Kristallpulver aus, welches abfiltriert und im Vakuum getrocknet wurde.
Ausbeute :
3.6 g (93 %), farblose Nadeln, Smp: 45 - 46 °C
R_{f} = 0.30 (EtOAc:MeOH = 1:1), [α]²⁵_{D} = - 1.97° (c = 1.17, H₂O)
^{**1**}**H-NMR** (D₂O): δ = 7.30 (dd; 1H, Th-H-3), 6.98 (dd; 1H, Th-H-4), 6.70 (dd; 1H, Th-H-5), 4.72 (m; 1H, CH), 4.30 (m; 1H, CH), 3.72 (s; 3H, OCH₃), 3.43 (dd; 1H, CH von -CH₂-), 3.33 (dd; 1H, CH von -CH₂-), 2.97 (m; 2H, -CH₂-), 1.36 (s; 9H, CH₃).
^{**13**}**C-NMR** (D₂O:d₆-DMSO = 7:1): δ = 173.73 (s; COO), 169.68 (s; COO), 139.39 (s; C), 128.90 (d; CH), 128.63 (d; CH), 126.87 (d; CH), 55.91 (d; CH), 54.53 (q; OCH₃), 50.67 (d; CH), 36.27 (t; -CH₂-), 31.95 (t; -CH₂-)

| C₁₂H₁₇BrN₂O₅S (381.25): | | | |
|---|---|---|---|
| Ber. | C 37.81 | H 4.49 | N 7.35 |
| Gef. | C 38.09 | H 4.81 | N 7.19 |

### 1.4. Herstellung von N-L-α-Aspartyl-L-(2-thienyl)alanin, Methylester (1)

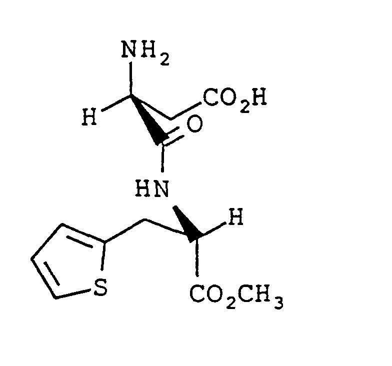

20 ml stark basischer Ionenaustauscher (Amberlite IRA-400) wurden über Nacht in gesättigter wässriger Natriumacetatlösung gerührt. Anschliessend wurde der Ionenaustauscher abfiltriert und mit Wasser neutral gewaschen.

Der so aktivierte Amberlite wurde mit einer Lösung von 3.1 g (8.10 mmol) 6 in 50 ml Wasser 30 min bei 45 °C gerührt. Der Feststoff wurde abfiltriert, mit 30 ml Wasser gewaschen und das Eluat, sowie die Waschlösungen im Vakuum stark eingeengt. Beim Abkühlen fielen farblose Nadeln aus, welche abfiltriert und getrocknet wurden.
Ausbeute :
1.8 g (74 %), farbloses Nadeln, Smp: 223 - 225 °C
R_{f}= 0.405 (EtOAc:MeOH=1:1), [α]²³_{D} = + 43°, (c = 0.71, HOAc)
^{**1**}**H-NMR** (D₂O): δ = 7.28 (dd; 1H, Th-H-3), 6.96 (dd; 1H, Th-H-4), 6.88 (dd; 1H, Th-H-5), 4.69 (m; 1H, CH), 4.15 (m; 1H, CH), 3.69 (s; 3H, OCH₃), 3.40 (dd; 1H, CH von -CH₂-), 3.28 (dd; 1H, CH von -CH₂-), 2.71 (dd; 1H, CH von -CH₂-), 2.60 (dd, 1H, CH von -CH₂-).
^{**13**}**C-NMR** (d₆-DMSO): δ = 172.29 (s; CO), 170.97 (s; CO), 170.70 (s; CO), 138.44 (s; C), 127.00 (d; CH), 126.70 (d; CH), 124.92 (d; CH), 53.70 (q; OCH₃), 52.15 (d; CH), 50.48 (d; CH), 37.41 (t; -CH₂-), 30.82 (t; -CH₂-).

| | | | |
|---|---|---|---|
| C₁₂H₁₆N₂O₅S (300.33): | | | |
| Ber. | C 47.99 | H 5.37 | N 9.21 |
| Gef. | C 47.70 | H 5.60 | N 9.33 |

### Beispiel 2

### Herstellung von N-L-α-Aspartyl-L-(3-thienyl)alanin, Methylester (2)

### 2.1. (S)-3-(3'-Thienyl)-alanin, Methylester, Hydrochlorid (7)

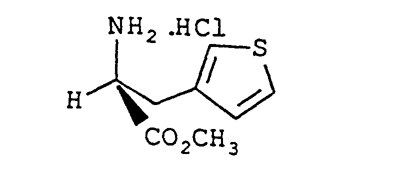

Durch eine Suspension von 4.00 g (23.4 mmol) (S)-3-(3'-Thienyl)-alanin in 150 ml wasserfreiem Methanol wurde bei Raumtemperatur 3 h ein starker Strom trockener Chlorwasserstoff geleitet.

Das Lösungsmittel wurde anschliessend bis zur Trockne abgedampft, der Rückstand mit 20 ml Methanol aufgenommen und mit Ether bis zur vollständigen Fällung versetzt. Das ausgefallene Produkt wurde abfiltriert und getrocknet.
Ausbeute :
4.6 g (89 %), bräunlicher Feststoff, Smp: 174 - 176 °C
[α]²³_{D} = + 5.3° (c = 0.76, MeOH)
^{**1**}**H-NMR** (D₂O): δ = 7.41 (dd; 1H, Th-H-4), 7.22 (d; 1H, Th-H-2), 6.93 (d; 1H, Th-H-5), 4.35 (t; 1H, α-CH), 3.77 (s; 3H, OCH₃), 3.25 (d; 2H, -CH₂-).
^{**13**}**C-NMR** (D₂O): δ = 172.6 (s; COO), 136.1 (s; C), 130.6 , 130.1, 127.4 (d; C), 56.2 (d; α-CH), 56.1 (q; OCH₃), 32.5 (t; -CH₂-).

### 2.2. L-β-tert.Butyl-N-carbobenzoxy-α-aspartyl-L-(3-thienyl) alanin, Methylester (8)

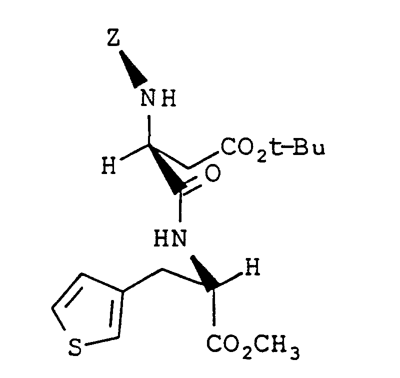

6.8 g (13.5 mmol) 4 wurden in 50 ml wasserfreiem DMF gelöst, bei 0 °C mit 3.00 g (13.5 mmol) 7 und 1.87 ml (13.5 mmol) Triethylamin versetzt und 3δbei 0 - 4°C gerührt.

Die Reaktionslösung wurde sodann zwischen 160 ml Eiswasser und 100 ml Ether verteilt, die wässrige Phase mehrmals mit Ether extrahiert und die vereinigten organischen Phasen mit gesättigter Natriumchloridlösung gewaschen. Die Etherlösung wurde mit Natriumsulfat getrocknet und das Lösungsmittel abdestilliert. Das so erhaltene Rohprodukt wurde über 220 g Kieselgel mit Petrolether:Ether (Gradient von 6:4 aus 1:2) geflasht.
Ausbeute :
5.2 g (79 %), farbloser Feststoff, Smp: 74 - 76°C
R_{f}= 0.364 (Petrolether:Ether=1:2), [α]²⁵_{D} = + 31.2°, (c = 1.1, CH₂Cl₂)
^{**1**}**H-NMR** (CDCl₃): δ = 7.30 - 7.25 (m; 5H, C₆H₅), 7.18 (m; 1H, Th-H), 6.98 (m; 1H, Th-H), 6.82 (m; 1H, Th-H), 5.90 (d; 1H, CONH), 5.06 (s; 2H, OCH₂), 4.73 (m; 1H, α-CH), 4.50 (m; 1H, α-CH), 3.66 (s; 3H, OCH₃), 3.08 (m; 2H, -CH₂-), 2.85 (dd; 1H, CH von -CH₂-), 2.50 (dd; 1H, CH von -CH₂-), 1.36 (s; 9H, CH₃).
^{**13**}**C-NMR** (CDCl₃): δ = 171.26 (s; CO), 171.07 (s; CO), 170.19 (s; CO), 155.89 (s; CONH), 136.01 (s; CH), 135.69 (s; C-3-Th), 128.49 (d; CH), 128.22 (d; CH), 128.17 (d; CH), 127.07 (d; CH), 125.86 (d; CH), 122.89 (d; CH), 81.76 (s; OC_{quart}), 67.15 (t; OCH₂), 52.88 (q; OCH₃), 52.25 (d; α-CH), 50.97 (d; α-CH), 37.15 (t; -CH₂), 32.12 (t; -CH₂-), 27.93 (q; CH₃).

### 2.3. N-L-α-Aspartyl-L-(3-thienyl)alanin, Methylester, Hydrobromid (9)

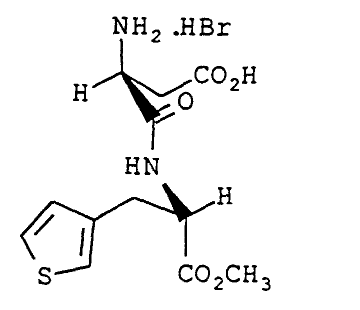

Eine Lösung von 5.0 g (10.2 mmol) 8 in 50 ml Eisessig wurde bei Raumtemperatur tropfenweise mit 15 ml (61.2 mmol) einer 4.1 M (33 %) Lösung von HBr in Eisessig versetzt und 2 h gerührt.

Die Lösungsmittel wurden anschliessend im Wasserstrahlvakuum abdestilliert und der ölige Rückstand mit 100 ml Ether digeriert. Dabei fiel ein farbloses Kristallpulver aus, welches abfiltriert und im Vakuum getrocknet wurde.
Ausbeute :
3.7 g (95 %), farblose Nadeln, Smp: 82 - 84 °C
R_{f} = 0.371 (EtOAc:MeOH = 1:1), [α]²⁵_{D} = +27° (c = 1.0, HOAc)
^{**1**}**H-NMR** (D₂O): δ = 7.39 (m; 1H, Th-H), 7.18 (m; 1H, Th-H), 6.98 (m; 1H, Th-H), 4.74 (m; 1H, α-CH), 4.28 (m; 1H, α-CH), 3.70 (s; 3H, OCH₃), 3.22 (dd; 1H, CH von -CH₂-), 3.15 (dd; 1H, CH von -CH₂-), 2.95 (m; 2H, -CH₂-).
^{**13**}**C-NMR** (D₂O): δ = 172.13 (s; COO), 171.84 (s; COO), 167.63 (s; CON-H), 137.30 (s; C-3-Th), 127.55 (d; CH), 125.87 (d; CH), 122.56 (d; CH), 54.43 (d; α-CH), 53.54 (q; OCH₃), 49.79 (d; α-CH), 35.31 (t; -CH₂-), 31.21 (t; -CH₂-).

| | | | |
|---|---|---|---|
| C₁₂H₁₇BrN₂O₅S. 0.40 H₂O (388.46): | | | |
| Ber. | C 37.10 | H 4.62 | N 7.21 |
| Gef. | C 37.15 | H 4.70 | N 7.08 |

### 2.4. N-L-α-Aspartyl-L-(3-thienyl)alanin, Methylester (2)

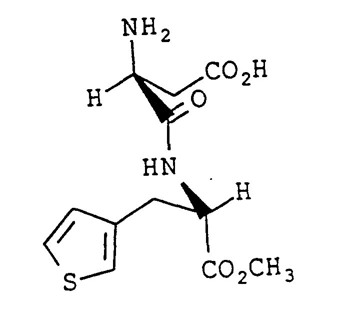

20 ml stark basischer Ionenaustauscher (Amberlite IRA-400) wurden über Nacht in gesättigter wässriger Natriumacetatlösung gerührt. Anschliessend wurde der Ionenaustauscher abfiltriert und mit Wasser neutral gewaschen.

Der so aktivierte Amberlite wurde mit einer Lösung von 3.5 g (9.20 mmol) 9 in 50 ml Wasser 30 min bei 45 °C gerührt. Der Feststoff wurde abfiltriert, mit 30 ml Wasser gewaschen, nochmals bei 45 °C mit 50 ml Wasser digeriert und das Eluat, sowie die Waschlösungen im Vakuum stark eingeengt. Beim Abkühlen fielen farblose Nadeln aus, welche abfiltriert und getrocknet wurden.
Ausbeute :
2.3 g (83 %), farbloses Nadeln, 234 - 235 °C
R_{f} = 0.408 (EtOAc:MeOH = 1:1), [α]²³_{D} =+30 °, (c = 1.0, HOAc)
^{**1**}**H-NMR** (D₂O): δ = 7.39 (m; 1H, Th-H), 7.17 (m; 1H, Th-H), 7.00 (m; 1H, Th-H), 4.70 (m; 1H, α-CH), 4.16 (m; 1H, α-CH), 3.70 (s; 3H, OCH), 3.20 (dd; 1H, CH von -CH₂-), 3.11 (dd; 1H, CH von -CH₂-), 2.72 (dd; 1H, CH von -CH₂-), 2.64 (dd, 1H, CH von -CH₂-).
^{**13**}**C-NMR** (D₂O): δ = 178.46 (s; CO), 175.68 (s; CO), 172.03 (s; CO), 139.08 (s; C-3-Th), 130.92 (d; CH), 129.20 (d; CH), 125.91 (d; CH), 56.55 (q; OCH₃), 55.69 (d; α-CH), 53.14 (d; α-CH), 39.57 (t; -CH₂-), 33.50 (t; -CH₂-).

| | | | |
|---|---|---|---|
| C₁₂H₁₆N₂O₅S. 0.70 H₂O (312.95): | | | |
| Ber. | C 46.06 | H 5.60 | N 8.95 |
| Gef. | C 46.06 | H 5.53 | N 8.81 |

### Beispiel 3

### N-L-α-Aspartyl-(L-[2-(5-methyl)-thienyl]alanin, Methylester (3)

### 3.1. 2-Hydroxymethyl-5-methyl-thiophen (10)

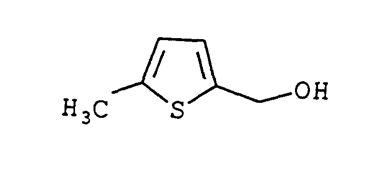

Eine Lösung von 50 ml (456 mmol) 5-Methylthiophen-2-carbaldehyd in 350 ml Ethanol wurde unter Eiskühlung und Rühren portionsweise mit 13.2 g (347 mmol) Natriumborhydrid versetzt. Nach vollständiger Zugabe wurden noch weitere 2 h gerührt und die Reaktionslösung anschliessend auf eine Mischung von 400 g Eis und 200 ml gesättigter Ammoniumchloridlösung gegossen. Nach Beeendigung der Gasentwicklung wurde die Lösung im Vakuum von Ethanol befreit. Der wässrige Rückstand wurde mehrmals mit Ether extrahiert, die vereinigten etherischen Phasen mit gesättigter Natriumchloridlösung gewaschen und über Natriumsulfat getrocknet. Das Lösungsmittel wurde abdestilliert. Zurück blieb ein öliger Rückstand, welcher bei einem Druck von 20 mbar destilliert wurde.
1. Fraktion: 55 - 100 °C 0.3 g
2. Fraktion: 101 - 99 °C 50.0 g
Das Produkt war auch bei Lagerung im Kühlschrank nicht stabil und sollte daher baldmöglichst umgesetzt werden.
Ausbeute:
50.3 g (86 %), farblose Flüssigkeit
R_{f} = 0.512 (Petrolether:Ether = 1:1).
^{**1**}**H-NMR** (CDCl₃): δ = 6.81 (d; 1H, CH), 6.63 (d; 1H, CH), 4.74 (s; 2H, -CH₂-), 2.50 (s; 3H, CH₃), 1.75 (bs; 1H, OH).
^{**13**}**C-NMR** (CDCl₃): δ = 141.70 (s; C), 140.24 (s; C), 125.54 (d; C), 124.82 (d; C), 59.86 (t; CH2), 15.39 (q; CH₃).

### 3.2. 2-Brommethyl-5-methyl-thiophen (11)

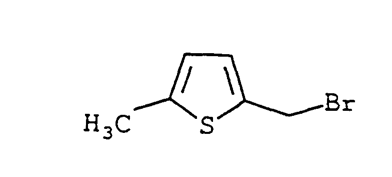

30 g (234 mmol) 10 in 300 ml wasserfreiem Ether wurden unter Eiskühlung mit einer Lösung von 149 g (550 mmol) Phosphortribromid in 350 ml wasserfreiem Ether innerhalb von 90 min versetzt. Nach 2 h Rühren bei Raumtemperatur wurde die Reaktionslösung auf 2 kg Eis gegossen und die organische Phase abgetrennt.

Die wässrige Phase wurde mit Natriumchlorid gesättigt und mehrmals mit Ether extrahiert. Die vereinigten organischen Phasen wurden mit Natriumsulfat getrocknet und das Lösungsmittel im leichtem Vakuum abgedampft.
Ausbeute: 38 g (95 %), braune Flüssigkeit.

Das Produkt neigt sehr stark zur Polymerisation und sollte daher nicht erwärmt, möglichst unter inerter Atmosphäre isoliert oder sofort weiterverarbeitet werden.
^{**1**}**H-NMR** (CDCl₃): δ = 6.85 (d; 1H, CH), 6.52 (d; 1H, CH), 4.65 (s; 2H, -CH₂-), 2.40 (s; 3H, CH₃).

### 3.3. 2-(Acetylamino)-3-[2'-(5'-methyl)-thienylmethyl]propandisäure, Diethylester (12)

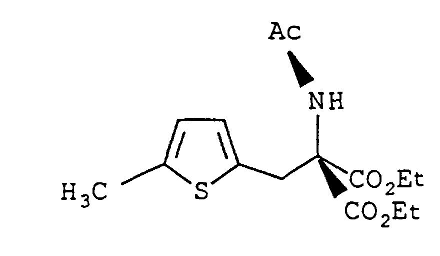

Zu einer Lösung von 3.56 g (155 mmol) Natrium in 200 ml trockenem Ethanol wurde bei Raumtemperatur 33.6 g (155 mmol) Diethyl-acetamidomalonat gegeben und solange gerührt, bis eine klare Lösung entstanden ist. Anschliessend wurden 29.0 g (152 mmol) 11 hinzugefügt und 3 h unter Rückfluss erhitzt. Das Lösungsmittel wurde im Vakuum abdestilliert, der Rückstand mit Ether/Wasser (3:1) aufgenommen und die wässrige Phase bis zur Erschöpfung mit Ether extrahiert. Die vereinigten organischen Phasen wurden mit gesättigter Natriumchlorid gewaschen, über Natriumsulfat getrocknet und der Ether abdestilliert.
Rohausbeute: 50 g
Da das Produkt mit Thiophenmethanol und dessen Zersetzungsprodukt verunreinigt war, wurde der Rückstand über 500 g Kieselgel mit Petrolether : Ether = 1:1 geflasht.
Ausbeute :
28 g, (55 %), Smp: 98 - 100°C
R_{f} = 0.4651 (Petrolether:Ether = 1:2).
^{**1**}**H-NMR** (CDCl₃): δ = 6.75 (bs; 1H, NH), 6.55 - 6.50 (m; 2H, Th-H-3,-4), 4.25 (q; 2H, OCH₂), 3.78 (s; 2H, -CH₂-), 2.40 (s; 3H, CH₃), 2.05 (s; 3H, Acetyl-CH₃), 1.30 (t; 6H, CH₃).
^{**13**}**C-NMR** (CDCl₃). δ = 168.87 (s; CONH), 166.97 (s; COO), 139.22 (s; C), 133.88 (s; C), 126.99 (d; CH), 124.60 (d; CH), 66.83 (s; C), 62.45 (t; OCH₂), 32.64 (t;-CH₂-), 22.80 (q; CH₃), 14.99 (q; CH₃), 13.76 (q; CH₃).

| | | | |
|---|---|---|---|
| C₁₅H₂₁NO₅S (327.40): | | | |
| Ber. | C 55.03 | H 6.47 | N 4.28 |
| Gef. | C 55.06 | H 6.43 | N 4.34 |

### 3.4. (R,S)-2-(Acetylamino)-3-[(5'-methyl)-2'-thienyl]propansäure (13)

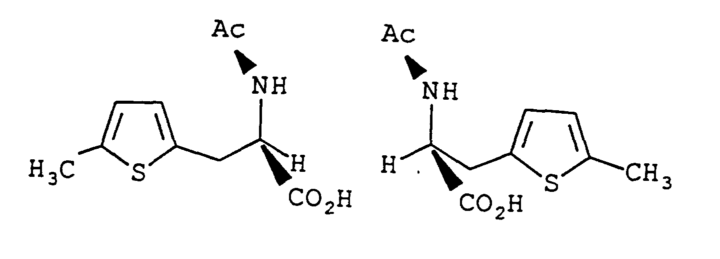

32.9 g (100.5 mmol) 12 wurden in 200 ml 10%iger Natronlauge suspendiert und 3 h unter Rückfluss erhitzt, wobei der Feststoff in Lösung ging. Anschliessend wurde mit konz. Salzsäure unter Eiskühlung bis pH = 1 angesäuert, wobei ein farbloser Niederschlag entstand. Es wurde erneut 3 h unter Rückfluss erhitzt.

Die Reaktionslösung wurde nach dem Abkühlen auf Raumtemperatur bis zu Erschöpfung mit Ethylacetat extrahiert, die vereinigten organischen Phasen mit gesättigter Natriumchlorid-Lösung gewaschen und mit Natriumsulfat getrocknet. Das Lösungsmittel wurde im Vakuum entfernt, der Rückstand im Vakuum getrocknet.
Ausbeute :
21.1 g (93 % d. Th.), farbloser Feststoff,
Smp: 165 - 166 °C
^{**1**}**H-NMR** (d₆-DMSO): δ = 8.22 (d; 1H, NH), 6.65 (d; 1H, CH), 6.59 (d; 1H, CH), 4.34 (m; 1H, α-CH), 3.15 (dd; 1H, CH von -CH₂-), 2.99 (dd; 1H, CH von -CH₂-), 2.37 (s; 3H, Ac-CH₃), 1.84 (s; 3H, CH₃).
^{**13**}**C-NMR** (d₆-DMSO): δ = 172.6 (s; COOH), 169.3 (s; CONH), 137.7 (s; C), 137.5 (s; C), 126.0 (d; CH), 124.8 (d; CH), 53.5 (d; α-CH), 31.3 (t; -CH₂-), 22.4 (q; Ac-CH₃), 14.8 (q; CH₃).

| | | | |
|---|---|---|---|
| C₁₀H₁₃NO₃S (227.29): | | | |
| Ber. | C 52.85 | H 5.76 | N 6.16 |
| Gef. | C 52.83 | H 5.76 | N 6.06 |

### 3.5. (S)-2-Amino-[2'-(5'-methyl)-thienyl]propansäure (14)

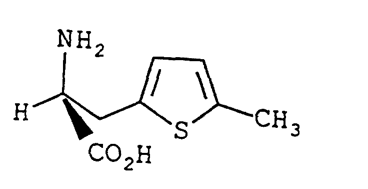

Eine Lösung von 13 g (157 mmol) Natriumcarbonat und 19.0 g (84.0 mmol) 13 in 800 ml Wasser wurde mit 2 N Salzsäure auf pH 7.5 - 8 eingestellt und mit 5.0 g Acylase versetzt. Die Suspension wurde 24 h bei Raumtemperatur gerührt.

Anschliessend wird mit konzentrierter Salzsäure auf pH 1-2 angesäuert und das denaturierte Enzym durch Filtration entfernt. Das Filtrat wurde erschöpfend mit Ethylacetat extrahiert, die vereinigten organischen Phasen mit gesättigter Natriumchloridlösung gewaschen und mit Natriumsulfat getrocknet. Nach Entfernen des Lösungsmittels im Vakuum blieb (R)-13 als ein bräunliches Kristallpulver zurück, welches im Vakuum getrocknet wurde.
Ausbeute:
8.9 g (94 %), farbloses Kristallpulver,
Smp: 164 °C, [α]²³_{D} = - 41 ° (c = 1.0, MeOH)

Die wässrige Phase wurde bis zur Trockne einrotiert, mit 70 ml Wasser aufgenommen und auf 250 ml aktivierten DOWEX 50 -Ionenaustauscher gegeben. Die Suspension wird 30 min bei Raumtemperatur gerührt, der Ionenaustauscher abfiltriert und solange mit Wasser gewaschen, bis das Eluat neutral reagierte. Anschliessend wurde der Ionenaustauscher mehrmals mit 1 M Ammoniaklösung digeriert, bis das Filtrat eine negative Ninhydrinprobe aufwies. Die vereinigten ammoniakalischen Filtrate wurden bis zur Trockene im Vakuum einrotiert und die zurückbleibende (S)-Aminosäure im Vakuum getrocknet.
Ausbeute:
7.7 g (100%), braunes Kristallpulver,
Smp: 252 - 253 °C
R_{f} = 0.281 (EtOAc:MeOH = 1:1)
[α]²³_{D}= - 3° (c = 1.0, 2N HCl)
Literatur : Smp: 253-5 °C
^{**1**}**H-NMR** (D₂O): δ = 6.66 (d; 1H, CH), 6.60 (d; 1H, CH), 3.67 (t; 1H, α-CH), 3.21 (d; 2H, -CH₂-), 2.35 (s; 3H, CH₃).
^{**13**}**C-NMR** (D₂O): δ = 180.4 (s; COOH), 142.7 (s; C), 138.5 (s; C), 129.6.1 (d; CH), 127.7 (d; CH), 58.0 (d; α-CH), 35.5 (t; -CH₂-), 16.9 (q; CH₃).

### 3.6. (S)-2-Amino-3-[2-(5-methyl)-thienyl]propansäure, Methylester, Hydrochlorid (15)

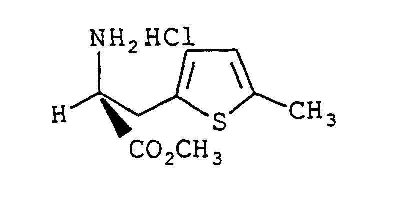

Durch eine Suspension von 3.1 g (16.7 mmol) 14 in 100 ml wasserfreiem Methanol wurde unter heftigem Rühren 3 h ein starker Strom von trockenem Chlorwasserstoff eingeleitet. Anschliessend wurde das Reaktionsgemisch bis zur Trockene eingeengt, der Rückstand mit Methanol aufgenommen und die Lösung mit Ether versetzt. Dabei fiel das Produkt als farbloser Feststoff aus, welcher abfiltriert und im Vakuum getrocknet wurde.
Ausbeute:
3.8 g (96%), farblose Nadeln, Smp: 170 - 172 °C
R_{f} = 0.739 (EtOAc: MeOH = 1:1)
[α]²³_{D} = + 17.5 ° (c = 1.0, MeOH)
¹H-NMR (D₂O): δ = 6.78 (d; 1H, CH), 6.70 (d; 1H, CH), 4.40 (t; 1H, α-CH), 3.44 (d, 2H, -CH₂-), 2.40 (s; 3H, CH₃).
^{**13**}**C-NMR** (D₂O): δ = 172.42 (s; CO), 144.16 (s; C), 134.93 (s; C), 131.26 (d; CH), 128.36 (d; CH), 56.60 (q; OCH₃), 56.47 (d; α-CH), 32.56 (t; -CH₂-), 17.05 (q; CH₃).

| | | | |
|---|---|---|---|
| C₉H₁₄ClNO₂S. 0.25 H₂O (240.24) | | | |
| Ber. | C 45.00 | H 6.08 | N 5.83 |
| Gef. | C 45.08 | H 5.91 | N 5.92 |

### 3.7. L-β-tert.Butyl-N-carbobenzoxy-α-aspartyl-L-[2-(5-methyl)thienyl]alanin, Methylester (16)

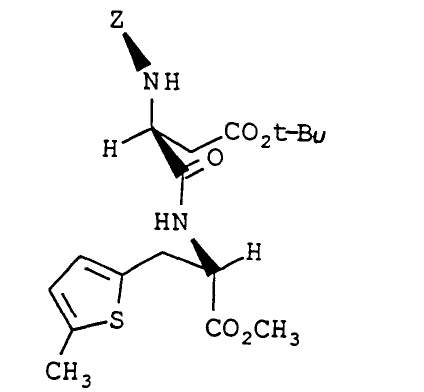

5.50 g (10.9 mmol) 4 werden in 50 ml wasserfreiem DMF gelöst und bei 0 °C mit 2.6 g (10.9 mmol) 15 und 1.51 ml (10.9 mmol) Triethylamin versetzt und 3 Tage bei 0 - 4°C gerührt.

Die Reaktionslösung wurde zwischen 80 ml Eiswasser und 50 ml Ether verteilt, die wässrige Phase mehrmals mit Ether extrahiert und die vereinigten organischen Phasen mit gesättigter Natriumchloridlösung gewaschen. Die Etherlösung wurde mit Natriumsulfat getrocknet und das Lösungsmittel abdestilliert. Das so erhaltene Rohprodukt wurde über 220 g Kieselgel mit Petrolether:Ether (Gradient von 6:4 aus 1:2) geflasht.
Ausbeute :
4.2 g (76 %), farbloser Feststoff, Smp: 56 - 57 °C
R_{f} = 0.462 (Petrolether:Ether = 1:2),
[α]²³_{D} = + 37.1 °, (c = 1.24, CH₂Cl₂)
^{**1**}**H-NMR** (CDCl₃): δ = 7.38 - 7.33 (m; 5H, C₆H₅), 7.08 (bd; 1H, CONH), 6.62 (d; 1H, Th-H), 6.56 (d; 1H, Th-H), 5.97 (d; 1H, CONH), 5.14 (s; 2H, OCH₂), 4.80 (m; 1H, α-CH), 4.58 (m; 1H, α-CH), 3.75 (s; 3H, OCH₃), 3.27 (m; 2H, -CH₂-), 2.90 (dd; 1H, CH von -CH₂-), 2.64 (dd; 1H, CH von -CH₂-), 2.41 (s; 3H, CH₃), 1.44 (s; 9H, CH₃).
^{**13**}**C-NMR** (CDCl₃): δ = 171.02 (s; CO), 170.91 (s; CO), 170.25 (s; CO), 155.95 (s; CONH), 139.31 (s; C-2-Th), 136.09 (s; C-1-Ph), 134.59 (s; C-5-Th), 128.55 (d; CH), 128.22 (d; CH), 128.05 (d; CH), 126.93 (d; CH), 125.06 (d; CH), 81.80 (s; OC_{quart}), 67.20 (t; OCH₂), 53.29 (q; OCH₃), 52.42 (d; α-CH), 51.04 (d; α-CH), 37.36 (t; -CH₂-), 32.09 (t; -CH₂-), 27.98 (q; CH₃), 15.23 (q; CH₃).

### 3.8. N-L-α-Aspartyl-[2-(5-methyl)thienyl)]alanin, Methylester, Hydrobromid (17)

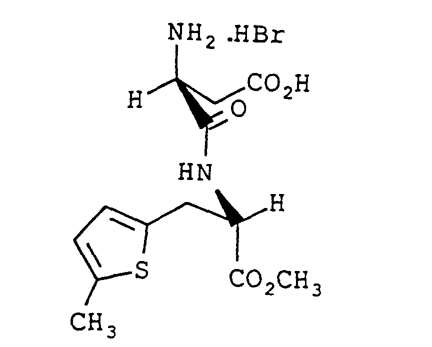

Eine Lösung von 4.0 g (7.93 mmol) **16** in 20 ml Eisessig wurde bei Raumtemperatur tropfenweise mit 11.6 ml (47.6 mmol) einer 4.1 M (33 %) Lösung von HBr in Eisessig versetzt und 2 h gerührt.

Die Lösungsmittel wurden im Wasserstrahlvakuum abdestilliert und der ölige Rückstand mit 100 ml Ether digeriert. Dabei fiel ein farbloses Kristallpulver aus, welches abfiltriert und im Vakuum getrocknet wurde.
Ausbeute :
3.0 g (96 %), beigefarbenes Kristallpulver,
Smp: 78 - 79 °C
R_{f} = 0.387 (EtOAc:MeOH = 1:1),
[α]²⁵_{D} = +26.7° (c = 0.75, HOAc)
^{**1**}**H-NMR** (D₂O): δ = 6.67 (d; 1H, Th-H), 6.62 (d; 1H, Th-H), 4.73 (m; 1H, α-CH), 4.32 (m; 1H, α-CH), 3.71 (s; 3H, OCH₃), 3.33 (m; 2H, -CH₂-), 3.00 (m; 2H, -CH₂-), 2.37 (s; 3H, CH₃).
^{**13**}**C-NMR** (D₂O): δ = 175.21 (s; CO), 175.01 (s; CO), 171.04 (s; CO), 142.88 (s; C-2-Th), 138.30 (s; C-5-Th), 129.71 (d; CH), 127.84 (d; CH), 57.14 (d; α-CH), 55.79 (q; OCH₃), 52.00 (d; α-CH), 37.48 (t; -CH₂-), 33.33 (t; -CH₂-), 16.96 (q; CH₃).

### 3.9. Herstellung von N-L-α-Aspartyl-(L-[2-(5-methyl)-thienyl]alanin, Methylester (3)

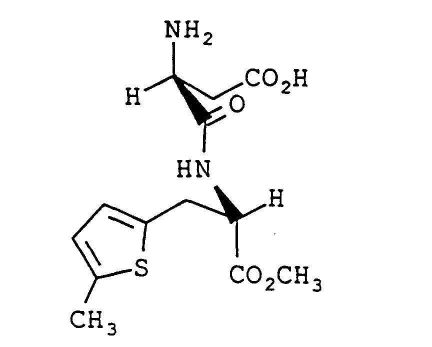

20 ml stark basischer Ionenaustauscher (Amberlite IRA-400) wurden über Nacht in gesättigter wässriger Natriumacetatlösung gerührt. Anschliessend wurde der Ionenaustauscher abfiltriert und mit Wasser neutral gewaschen.

Der so aktivierte Amberlite wurde mit einer Lösung von 2.80 g (7.10 mmol) 17 in 50 ml Wasser 30 min bei 45 °C gerührt. Der Feststoff wurde abfiltriert, mit 30 ml Wasser gewaschen und das Eluat sowie die Waschlösungen im Vakuum stark eingeengt. Beim Abkühlen fielen farblose Nadeln aus, welche abfiltriert und getrocknet wurden.
Ausbeute :
1.6 g (72 %), farbloses Nadeln, Smp: 234 - 235 °C
R_{f} = 0.428 (EtOAc:MeOH = 1:1),
[α]²⁵_{D} = + 45 °, (c = 0.75, HOAc)
^{**1**}**H-NMR** (D₂O): δ = 6.68 (d; 1H, Th-H), 6.63 (d; 1H, Th-H), 4.67 (m; 1H, α-CH), 4.19 (m; 1H, α-CH), 3.72 (s; 3H, OCH₃), 3.32 (dd; 1H, CH von -CH₂-), 3.22 (dd; 1H, CH von -CH₂-), 2.75 (dd; 1H, CH von -CH₂-), 2.63 (dd, 1H, CH von -CH₂-Hz).
^{**13**}**C-NMR** (D₂O): δ = 178.49 (s; CO), 175.21 (s; CO), 172.09 (s; CO), 142.87 (s; C-2-Th), 138.37 (s; C-5-Th), 129.66 (d; CH), 127.81 (d; CH), 57.03 (d; α-CH), 55.74 (q; OCH₃), 53.21 (d; α-CH), 39.62 (t; -CH₂-), 33.45 (t; -CH₂-), 16.90 (q; CH₃).

## Patentansprüche

1. L-Aspartyl-L-thienylalaninmethylester der Formel 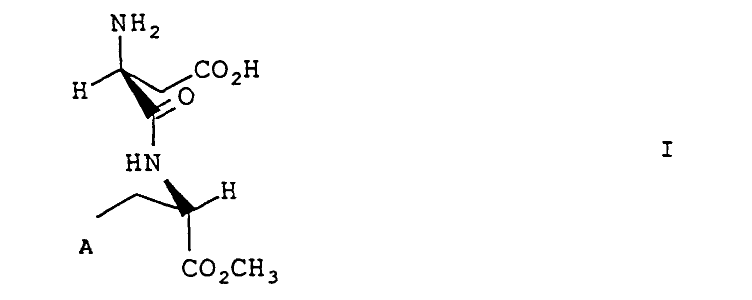 in der A einen Rest der Formel 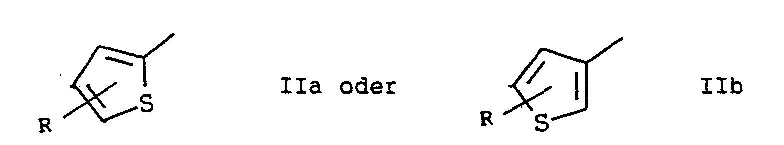 bedeutet, worin R H oder CH₃ sein kann.

2. Verbindungen der Formel I, nach Anspruch 1,
dadurch gekennzeichnet, dass A einen 2'-Thienyl-, 3'-Thienyl- oder 2'-(5'-Methyl)thienylrest bedeutet.

3. Verfahren zur Herstellung von Verbindungen der Formel 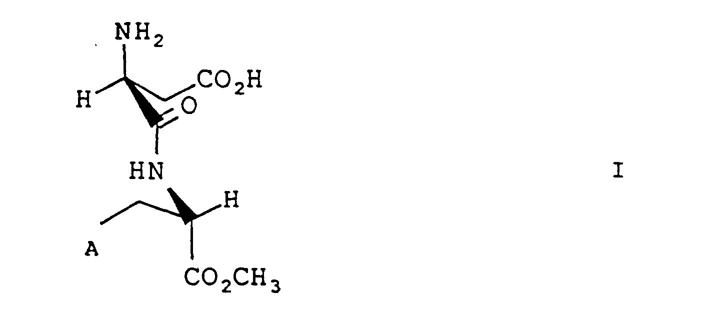 in der A einen Rest der Formel 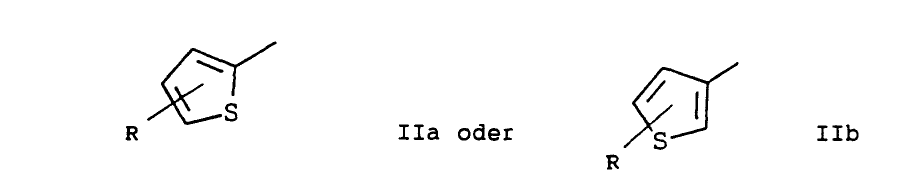 bedeutet, worin R H oder CH₃ sein kann, dadurch gekennzeichnet, dass eine Verbindung der Formel 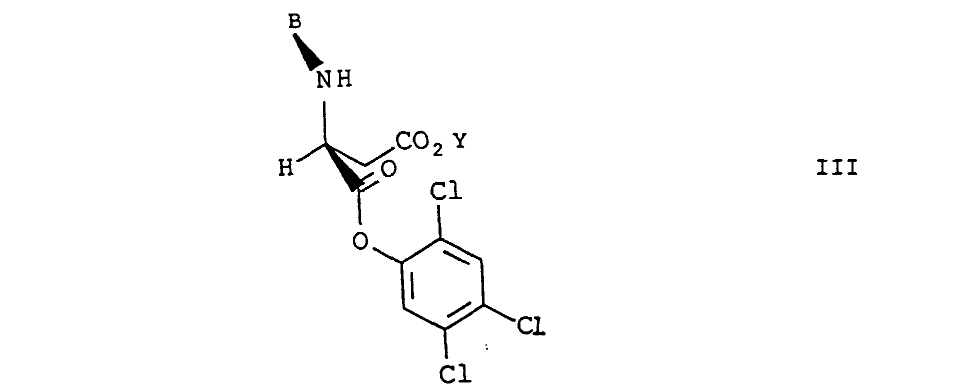 in der B eine Aminoschutzgruppe und Y eine Carboxylschutzgruppe bedeutet, in Gegenwart eines Säurefängers mit einer Verbindung der Formel 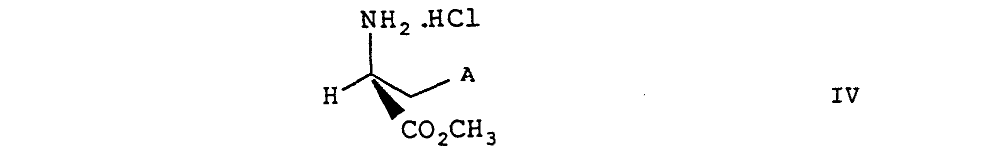 in der A obige Bedeutung hat, zu einer Verbindung der Formel 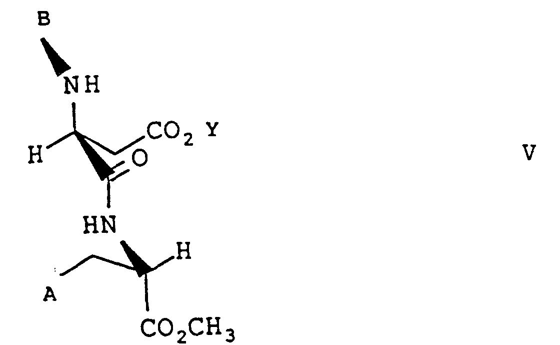 umgesetzt wird, in der A, B und Y obige Bedeutung haben, worauf nach Abspaltung der Schutzgruppen auf acidolytischem Weg, die Isolierung der entsprechenden Verbindung der Formel I aus dem so gewonnenen Salz mittels einer Base erfolgt.

4. Verwendung von Verbindungen der Formel 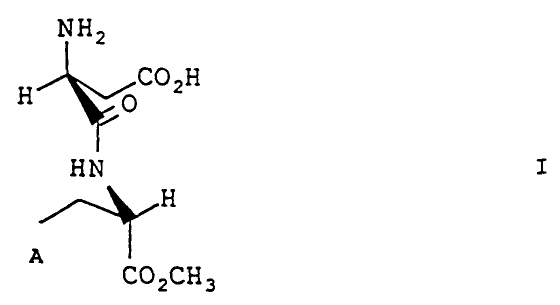 in der A einen Rest der Formel 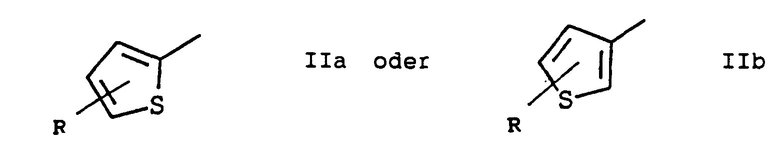 bedeutet, worin R H oder CH3 sein kann, als künstlicher Süssstoff.

## Claims

1. The methyl ester of L-aspartyl-L-thienylalanine of the formula 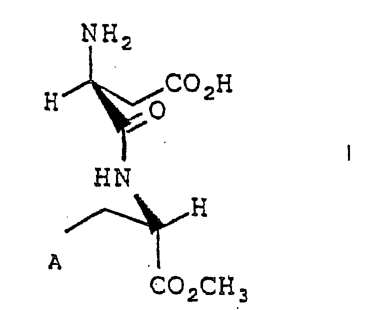 wherein A represents a radical of the formula 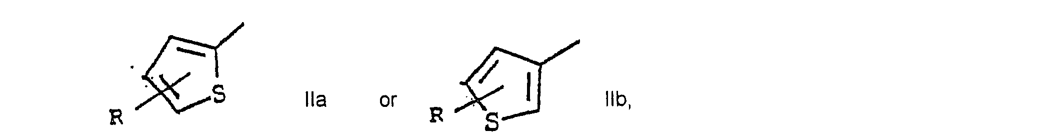 wherein R is selected from H and CH₃.

2. The compounds of formula I according to claim 1, characterized in that A represents a 2'-thienyl radical, a 3'-thienyl radical, or a 2'-(5'-methyl)thienyl radical.

3. A process for preparing compounds of the formula 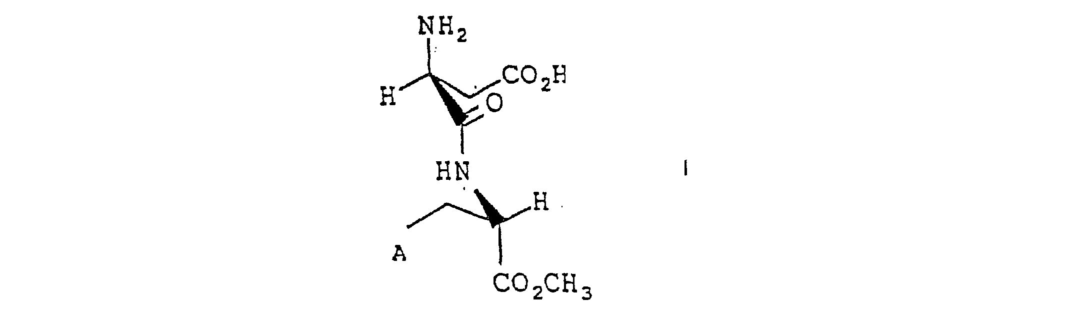 wherein A represents a radical of the formula 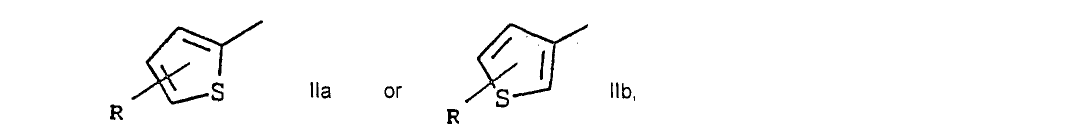 wherein R is selected from H and CH₃, characterized by reacting a compound of the formula 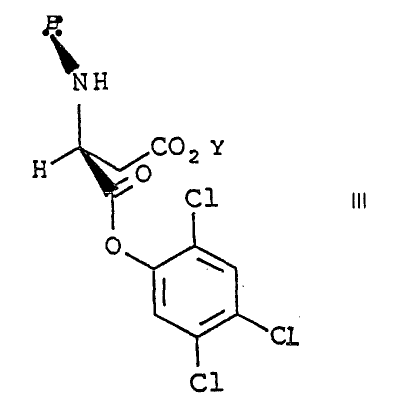 wherein B is an amino protecting group and Y is a carboxyl protecting group, with a compound of the formula 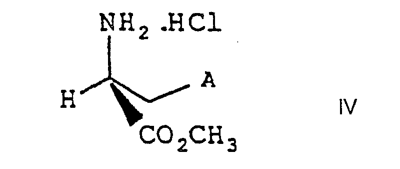 wherein A is as defined above, in the presence of an acid scavenger to give a compound of the formula 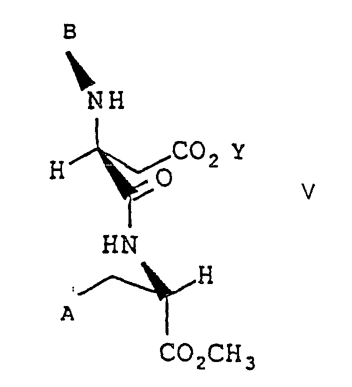 wherein A, B and Y are as defined above, followed by acidolytical cleaving the protecting groups and isolating the corresponding compound of the formula I from the salt thus obtained by use of a base.

4. The use of compounds of the formula 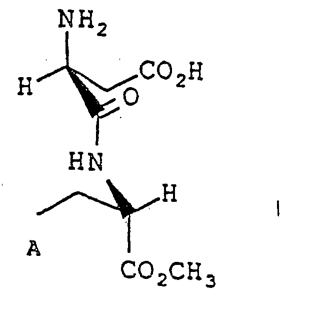 wherein A represents a radical of the formula 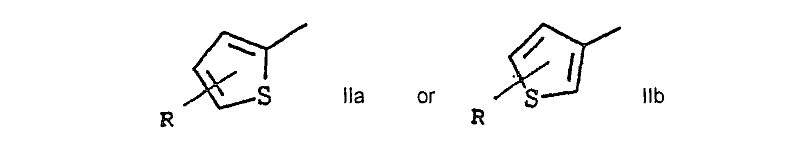 wherein R is selected from H and CH₃, as a synthetic sweetening agent.

## Revendications

1. Ester méthylique de L-aspartyl-L-thiénylalanine de la formule 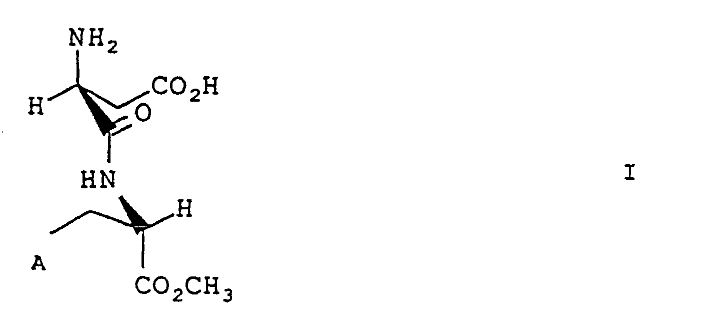 dans laquelle A est un reste de la formule 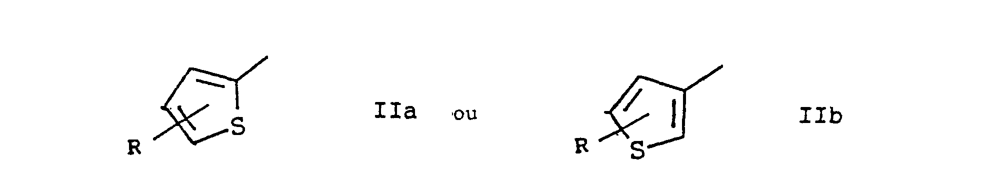 où R peut être H ou CH₃.

2. Composés de la formule I selon la revendication 1, caractérisés en ce que A est un reste de 2'-thiényle, de 3'-thiényle ou de 2'-(5'méthyl)thiényle.

3. Procédé de fabrication de composés de la formule 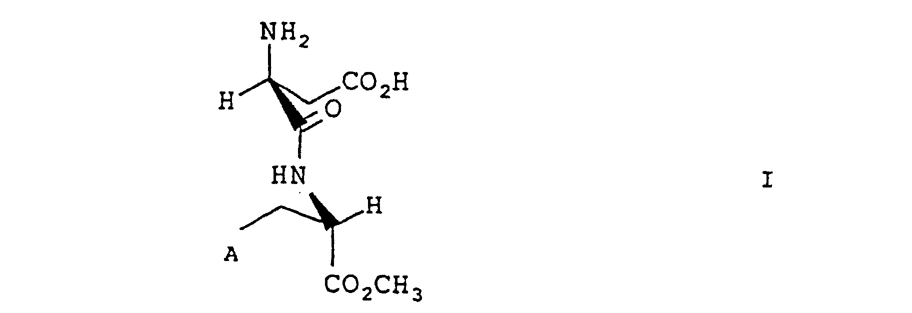 dans laquelle A est un reste de la formule 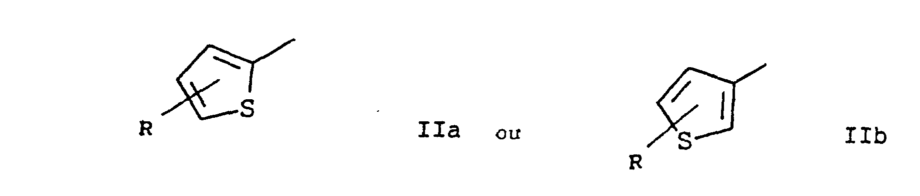 où R peut être H ou CH₃,
caractérisé en ce qu'un composé de la formule 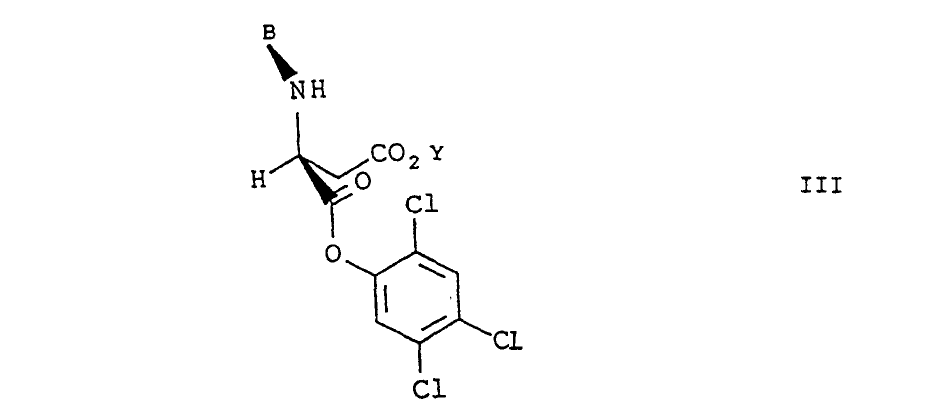 dans laquelle B est un groupe de protection amino et Y un groupe de protection carboxyle, lequel composé, en présence d'un inhibiteur d'acide associé à un composé de la formule 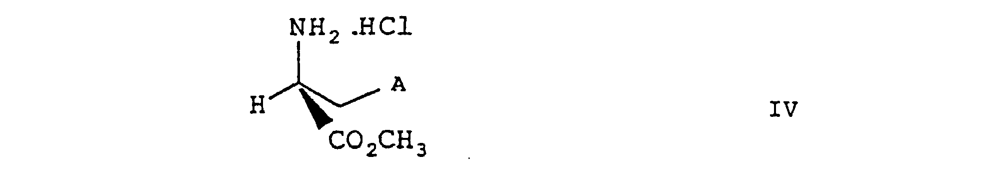 dans laquelle A a la signification mentionnée précédemment, est transformé en un composé de la formule 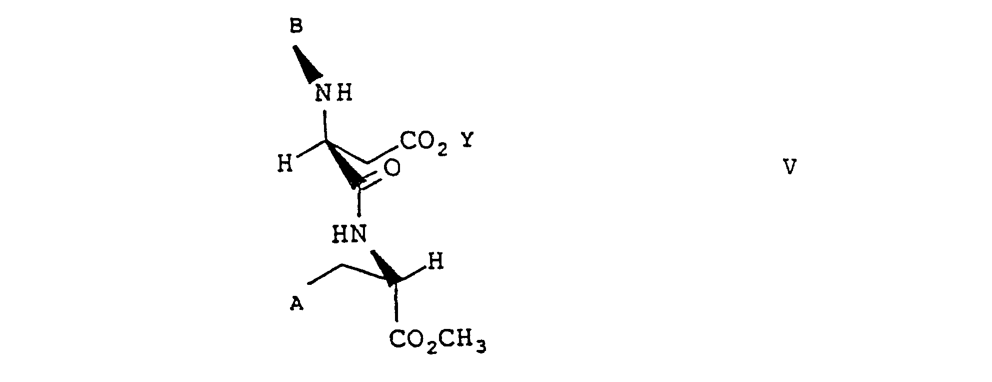 dans laquelle A, B et Y ont la signification mentionnée précédemment, à la suite de quoi, après séparation des groupes de protection par acidolyse, l'isolement du composé correspondant de la formule I, à partir du sel ainsi obtenu, se produit à l'aide d'une base.

4. Utilisation de composés de la formule 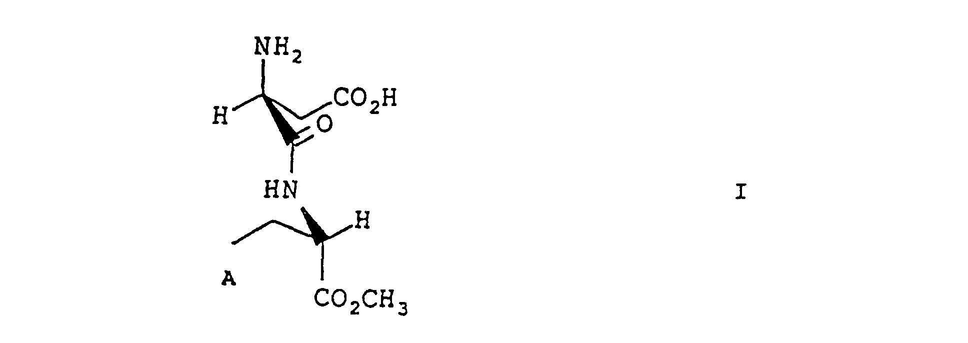 dans laquelle A est un reste de la formule 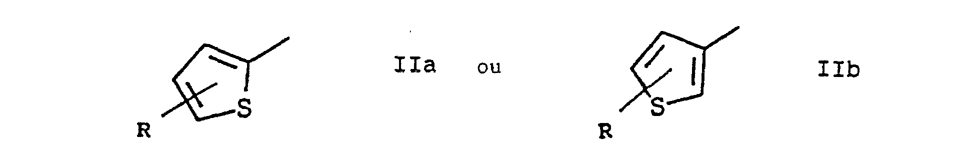 où R peut être H ou CH₃ en tant qu'édulcorant artificiel.
